Europäisches Patentamt

European Patent Office

Office européen des brevets

(11) Veröffentlichungsnummer: **0 287 086**
**A2**

(12) **EUROPÄISCHE PATENTANMELDUNG**

(21) Anmeldenummer: 88105920.8

(22) Anmeldetag: 14.04.88

(51) Int. Cl.⁴: **C07C 45/67 , C07C 47/21**

(30) Priorität: 16.04.87 DE 3713127

(43) Veröffentlichungstag der Anmeldung:
19.10.88 Patentblatt 88/42

(84) Benannte Vertragsstaaten:
CH DE FR GB LI NL

(71) Anmelder: **Consortium für elektrochemische Industrie GmbH**
**Zielstattstrasse 20**
**D-8000 München 70(DE)**

(72) Erfinder: **Staiger, Gerhard, Dr. Dipl.-Chem.**
**Staudingerstrasse 65**
**D-8000 München 83(DE)**

(54) **Verfahren zur Herstellung von Tiglinaldehyd.**

(57) Einstufiges Verfahren zur Herstellung von Tiglinaldehyd durch katalytische Isomerisierung von Ethylacrolein in flüssiger oder gasförmiger Phase bei Normaldruck in einem Temperaturbereich zwischen 50° C und 250° C unter Verwendung von Metallsalzen und/oder Metallkomplexen von Metallen der 8. Nebengruppe des PSE, vorzugsweise Palladiumalkanoate, gegebenenfalls unter Zusatz von Phosphinverbindungen und nach Auftragung des Katalysators auf ein Trägermaterial, wie z. B. Aktivkohle.

EP 0 287 086 A2

## Verfahren zur Herstellung von Tiglinaldehyd

Die Erfindung betrifft ein Verfahren zur Herstellung von Tiglinaldehyd aus Ethylacrolein.

Tiglinaldehyd (trans-2-Methyl-but-2-en-1-al) ist ein wichtiges Ausgangsmaterial zur Herstellung von Terpenen, von Tiglinsäure und von Riechstoffen.

In Liebigs Ann. 494, 273 (1932) wird beschrieben, daß man durch Behandlung von α-Ethylacrolein mit Calciumchlorid und anschließender Destillation Tiglinaldehyd erhält. Eine Umwandlung von 2-Alkylacroleinen in die entsprechenden 2-Methyl-2-alkenale kann man auch erreichen, wenn man zunächst die jeweiligen 2-Alkylacroleindialkylhydrazone herstellt, diese dann mit katalytischen Mengen starker Säuren in die Dialkylhydrazone der entsprechenden 2-Methyl-2-alkenale umlagert und zuletzt aus den Hydrazonen die 2-Methyl-2-alkenale durch Hydrolyse freisetzt [Zh. Org. Khim. 5, 1183 (1969)]. Man benötigt mit diesem Verfahren 3 Reaktionsschritte zur Synthese des Tiglinaldehyds. Das Dialkylhydrazon fällt im letzten Reaktionsschritt als Salz an und muß daher mit Alkali unter Neutralsalzbildung freigesetzt werden.

Tiglinaldehyd läßt sich auch durch eine gekreuzte Aldolkondensation aus Acetaldehyd und Propionaldehyd herstellen (Th. B. Green, W.T. Hickingbottom J. Chem. Soc. 1975, S. 3262) Nach dieser Methode entsteht jedoch eine große Zahl von Nebenprodukten unter mäßiger Ausbeute an Tiglinaldehyd.

In der DE-OS 34 21 809 (US-A 4,599,458) wird ein Verfahren beschrieben mittels dessen 2-Methyl-2-alkenale unter Katalyse von Zeolith, Aluminiumsilikaten und/oder Aluminiumphosphaten aus 2-Alkylacroleinen hergestellt werden. Neben der aufwendigen Herstellung des katalytisch aktiven Zeoliths besteht ein weiterer Nachteil dieses Verfahrens in der geringen Isomerisierungsrate, selbst bei Temperaturen von 400° C . Dies erfordert eine aufwendige Trennung des Produkts vom Edukt. Die hohe Isomerisierungstemperatur führt überdies zur Abscheidung von Koks auf dem Katalysator und damit zu dessen Desaktivierung In einem ähnlichen Verfahren nach der EP-A 1 168 761 wird Tiglinaldehyd aus Dihydropyranen, d. h. Dimeren der Alkylacroleine in Gegenwart von Zeolith, Aluminiumsilikat und/oder Aluminiumphosphat hergestellt.

In der EP-A1 171216 (US-A 4,605,779) wird ein Verfahren zur Isomerisierung von Ethylacrolein zu Tiglinaldehyd, unter Verwendung eines Hydrierkatalysators, bevorzugt in Gegenwart von Wasserstoff, beschrieben. Die Isomerisierung erfolgt mit diesem Verfahren sowohl in der Gasphase als auch in der flüssigen Phase. Als Katalysator wird bevorzugt 5 % Palladium auf Aktivkohle oder 0,5 % Palladium auf Aluminiumoxid verwendet. Als Nebenreaktion tritt bei diesem Verfahren, aufgrund der Anwesenheit von $H_2$-Gas, die Hydrierung des Ethylacroleins zum 2-Methylbutanal auf. Diese Nebenreaktion versucht man durch eine umständliche Desaktivierung des Katalysators mit Schwefelverbindungen, wie z. B. Natriumdithionit zu unterdrücken. Die Hydrierung des Ethylacroleins bringt neben der Ausbeuteverminderung auch den Nachteil einer aufwendigen destillativen Reinigung des Rohprodukts mit sich. Ohne $H_2$-Gaszusatz ist eine deutliche Reduzierung der Ausbeute an Tiglinaldehyd bei diesem Verfahren zu beobachten.

Aufgabe der Erfindung war es daher, ein Verfahren zur katalytischen Isomerisierung von Ethylacrolein zu Tiglinaldehyd zu entwickeln, das in einem Reaktionsschritt mit hoher Spezifität und in guten Ausbeuten zum Tiglinaldehyd führt, ohne die oben erwähnten Nachteile wie aufwenige Katalysatorbereitung, hohe Reaktionstemperaturen und Nebenproduktbildung aufzuweisen.

Gegenstand der Erfindung ist ein Verfahren zur Herstellung von Tiglinaldehyd durch Isomerisierung von Ethylacrolein in flüssiger oder gasförmiger Phase unter Normaldruck in einem Temperaturbereich zwischen 50° C und 250° C dadurch gekennzeichnet, daß die Isomerisierung unter Katalyse eines Metallsalzes und/oder Metallkomplexes eines Elements der 8. Nebengruppe des Periodensystems nach Mendelejew erfolgt.

Ausgangsmaterial des Verfahrens ist Ethylacrolein, das sich nach bekannten Verfahren, wie z. B. in der DE-OS 31 06 557, aus n-Butanol und wässrigem Formaldehyd in Gegenwart von sekundären Aminen und Carbonsäuren herstellen läßt. Das erfindungsgemäße Verfahren erfordert kein hochreines Ethylacrolein als Edukt, d. h. Restbestandteile an n-Butanal, Formaldehyd und Wasser können toleriert werden, so daß auch das Rohprodukt der Aldolkondensation zur somerisierung eingesetzt werden kann. Als Isomerisierungskatalysator werden im erfindungsgemäßen Verfahren Metallsalze und/oder Metallkomplexe von Metallen der 8. Nebengruppe des Periodensystems, insbesondere der Platinmetalle, vorzugsweise Metallsalze und/oder Metallkomplexe von Rhodium und Palladium, insbesondere Palladiumalkanoate von Monocarbonsäuren mit 1 bis 8 C-Atomen, wie z. B. Palladiumacetat, Palladiumpropionat verwendet. Der Katalysator kann zur Isomerisierung direkt oder auf einem Trägermaterial aufgetragen eingesetzt werden. Als Katalysatorträgermaterial eignet sich Kieselgel, Aluminiumoxid, vorzugsweise Aktivkohle, das in Pulverform oder als poröses Granulat eingesetzt wird.

Zur Erhöhung der Katalysatorstabilität und der Selektivität der Isomerisierungsreaktion ist es vorteilhaft, aromatische oder aliphatische Phosphinverbindungen wie z. B. Triphenylphosphin, Bis-(diphenylphosphino)-ethan und/ oder Tributylphosphin dem Katalysatorsystem zuzusetzen.

Die Isomerisierungsreaktion wird unter Normaldruck in einem Temperaturbereich zwischen 50° C und 250° C in flüssiger Phase oder in der Gasphase durchgeführt.

Bei der Reaktion in der flüssigen Phase unter Normaldruck beträgt die Reaktionstemperatur vorzugsweise zwischen 80° C und 110° C. Das Edukt (Ethylacrolein) wird bei der Reaktion in flüssiger Phase entweder rein oder gelöst in einem inerten Lösungsmittel eingesetzt. Als inerte Lösungsmittel können aliphatische und aromatische Kohlenwasserstoffe, Chlorkohlenwasserstoffe, Ether und Alkohole wie Toluol, Xylol, n-Octan, Chloroform, n-Butanol eingesetzt werden. Bei der Reaktion in flüssiger Phase beträgt die Konzentration des Katalysators bezogen auf Ethylacrolein vorzugsweise 1 bis 5 Mol%.

Die Isomerisierung in der Gasphase wird vorzugsweise bei 150° C bis 200° C unter Normaldruck durchgeführt. Das gasförmige Ethylacrolein kann gegebenenfalls unter Inertgaszusatz mit dem Isomerisierungskatalysator in Kontakt gebracht werden. Als Inertgas kann z. B. Stickstoff oder Argon eingesetzt werden. Die Katalysatorkonzentration in der Gasphase bezogen auf Ethylacrolein und den Durchsatz pro Stunde beträgt ebenfalls 1 bis 5 Mol%. Da bei der Gasphasenreaktion die Aktivität und Selektivität des Katalysators konstant bleibt erhält man durch Verlängerung der Reaktionszeit im Fall des auf Aktivkohle aufgebrachten Palladiumacetats ein Molverhältnis zu Ethylacrolein von mehr als 2000 : 1.

Unter den hier beschriebenen Bedingungen erhält man mit dem erfindungsgemäßen Verfahren Tiglinaldehyd in sehr guten Ausbeuten und außer wenigen Prozent Ethylacrolein, das aber destillativ einfach abtrennbar ist, ohne Bildung von Verunreinigungen und Nebenprodukten, vor allem ohne Bildung von Hydrierprodukten.

Beispiel 1

Man erwärmt ein Gemisch aus 1 g (12 mmol) Ethylacrolein, 140 mg (0,34 mmol) 1.2-Bis-(diphenylphosphino)-ethan, 100 mg (0,17 mmol) Bis-(dibenzylidenaceton)-palladium-(0) sowie 50 mg Wasser unter Rühren auf Rückflußtemperatur. Nach 20 h Reaktionszeit zeigt die gaschromatographische Analyse eine Gemischzusammensetzung von 83,2 % Tiglinaldehyd, 13,5 % Ethylacrolein sowie 3,3 % nicht identifizierte Nebenprodukte.

Beispiel 2

Ein Gemisch aus 5 g (60 mmol) Ethylacrolein, 1,57 g (6,0 mmol) Triphenylphosphin, 0,675 g (3,0 mmol) Palladiumacetat sowie 1,24 g (12 mmol) Benzonitril und 250 mg Wasser wird in der Kälte zusammengegeben und dann unter Rühren auf Rückflußtemperatur erhitzt. Nach 20 h Reaktionszeit besteht der flüchtige Anteil der Mischung aus 76,0 % Tiglinaldehyd, 19,4 % Benzonitril, 1,1 % Ethylacrolein sowie 3,5 % nicht identifizierte Nebenprodukte.

Beispiel 3

Ein Gemisch aus 25 g (300 mmol) Ethylacrolein, 3,94 g (15,0 mmol) Triphenylphosphin und 1,685 g (7,5 mmol) Palladiumacetat wird 20 Stunden lang auf Rückflußtemperatur erhitzt. Das resultierende Gemisch besteht zu 94,2 % aus Tiglinaldehyd, 1,6 % aus Ethylacrolein und zu 4,2 % aus Nebenprodukten.

Beispiel 4

1. Katalysatorbereitung

Zu einer Lösung von 1,0 g (4,5 mmol) Palladiumacetat in 70 ml Methylenchlorid gibt man 65 g Aktivkohle, (Korndurchmesser 2,5 mm). Die Aktivkohle, die die Lösung völlig aufgesaugt hat, wird zunächst im Luftstrom, dann im Hochvakuum (p < 0,1 Pa) bei 60° C getrocknet. Danach fügt man diese Aktivkohle zu einer Lösung von 2,35 g (9,0 mmol) Triphenylphosphin in 50 ml Toluol. Das Toluol wird danach zunächst im Luftstrom, dann im Hochvakuum bei 60° C von der Aktivkohle soweit als möglich (bis zur Gewichtskonstanz) entfernt. Anschließend wird die so vorbehandelte Aktivkohle nochmals zu einer Lösung von 1,0g (4,5mmol) Palladiumacetat in 60 ml Methylenchlorid gegeben und wie oben beschrieben getrocknet.

2. Isomerisierung von Ethylacrolein

In ein senkrecht stehendes, beheizbares Reaktorrohr werden 70 g des oben beschriebenen Palladium/Aktivkohlekatalysators eingefüllt. Auf diese Aktivkohle schichtet man noch etwa 10 cm hoch Glaskugeln. In dieser Zone verdampft das über eine Pumpe zudosierte Ethylacrolein, bevor es über den Festbettkatalysator strömt. Im Anschluß an die Katalysatorfüllung wird das Isomerisierungsprodukt in einem Kühler kondensiert und in einem Vorratsgefäß aufgefangen. Bei der erstmaligen Inbetriebnahme wird der Reaktor auf 150° C aufgeheizt und 0,3 ml Ethylacrolein pro Minute zudosiert. Dabei erwärmt sich das Katalysatorbett auf über 180° C. Nachdem diese primäre Wärmetönung abgeklungen ist, hält man die Reaktionstemperatur durch externe Heizung auf 170° C. Bei einer Dosiergeschwindigkeit von 0,3 ml/min erhält man in 75 %iger Ausbeute ein Produktgemisch, das zu 85 % Tiglinaldehyd enthält. Mit einer Katalysatorfüllung (2 g Palladiumacetat, 65 g Aktivkohle) lassen sich mindestens 3,0 kg Ethylacrolein umsetzen, ohne daß eine Verminderung der Katalysatoraktivität festzustellen wäre. Die Nebenbestandteile des Reaktionsgemisches, vor allem Ethylacrolein, lassen sich durch einfache Fraktionierung abtrennen.

## Ansprüche

1. Verfahren zur Herstellung von Tiglinaldehyd durch Isomerisierung von Ethylacrolein in flüssiger oder gasförmiger Phase unter Normaldruck in einem Temperaturbereich zwischen 50° C und 250° C dadurch gekennzeichnet, daß die Isomerisierung unter Katalyse eines Metallsalzes und/oder Metallkomplexes eines Elements der 8. Nebengruppe des Periodensystems erfolgt.

2. Verfahren nach Anspruch 1, dadurch gekennzeichnet, daß als Katalysator Metallsalze und/oder Metallkomplexe der Platinmetalle eingesetzt werden.

3. Verfahren nach Anspruch 1, dadurch gekennzeichnet, daß als Katalysator Metallsalze und/oder Metallkomplexe von Rhodium und Palladium eingesetzt werden.

4. Verfahren nach Anspruch 1, dadurch gekennzeichnet, daß als Katalysator Palladiumalkanoate von Monocarbonsäuren mit 1 bis 8 C-Atomen eingesetzt werden.

5. Verfahren nach Anspruch 1, 2, 3 und 4, dadurch gekennzeichnet, daß der Katalysator auf einem Katalysatorträger aufgetragen ist.

6. Verfahren nach Anspruch 1, 2, 3, 4 und 5, dadurch gekennzeichnet, daß zur Cokatalyse zusätzlich Phosphinverbindungen eingesetzt werden.